# EUROPÄISCHE PATENTSCHRIFT

(11) **EP 1 012 188 B1**
(45) Veröffentlichungstag und Bekanntmachung des Hinweises auf die Patenterteilung: **18.08.2004**
(21) Anmeldenummer: 98950026.9
(22) Anmeldetag: 11.09.1998
(51) Int. Cl.: C07K 14/605, A61K 38/22, A61P 3/10, A61P 3/04, A61P 3/06

(54) **ZUSAMMENSETZUNG ZUR THERAPIE VON DIABETES MELLITUS UND FETTSUCHT**
COMPOSITION FOR TREATING DIABETES MELLITUS AND OBESITY
COMPOSITION POUR LE TRAITEMENT DU DIABETE SUCRE ET DE L'OBESITE

(30) Priorität: 12.09.1997 DE 19740081; 23.12.1997 DE 19757739; 11.03.1998 DE 19810515
(43) Veröffentlichungstag der Anmeldung: 28.06.2000
(73) Patentinhaber: Pharis Biotec GmbH, 30625 Hannover (DE)
(72) Erfinder: FORSSMANN, Wolf, Georg, D-30625 Hannover (DE); RICHTER, Rudolf, D-30177 Hannover (DE); ADERMANN, Knut, D-30177 Hannover (DE); MEYER, Markus, D-30625 Hannover (DE)
(74) Vertreter: Meyers, Hans-Wilhelm, Dr.rer.nat.Dipl.-Chem.
(86) Internationale Anmeldenummer: PCT/EP1998/005804
(87) Internationale Veröffentlichungsnummer: WO 1999/014239

(56) Entgegenhaltungen:
- WO-A-91/11457
- WO-A-93/18786
- WO-A-95/31214
- WO-A-97/23461
- WO-A-97/24334
- WO-A-97/31943
- WO-A-98/37894
- US-A- 5 140 102
- SCHMIDTLER ET AL: "GLP-1(7-36) amide, -(1-37) and -(1-36) amide: Potent cAMP-Dependent Stimuli of Rat Parietal Cell Function" AMERICAN JOURNAL OF PHYSIOLOGY, Bd. 260, Nr. 6-I, Juni 1991, Seiten g940-g950, XP002093090
- DUNDORE ET AL: "Effects of Nucleotide Cyclase Activators on the Depressor Response to Selective Low Km Cyclic AMP Phosphodiesterase Inhibitors in Rats" DRUG DEVELOPMENT RESEARCH, Bd. 23, Nr. 2, 1991, Seiten 171-180, XP002093091
- FISCH ET AL: "Synergistic Interaction of Adenylate Cyclase Activators and Nitric Oxide Donor SIN-1 on Platelet Cyclic AMP" EUROPEAN JOURNAL OF PHARMACOLOGY, MOLECULAR PHARMACOLOGY SECTION, Bd. 289, Nr. 3, 1995, Seiten 455-461, XP002093092

### SEQUENZPROTOKOLL

(1) ALLGEMEINE ANGABEN:
   (i) ANMELDER:
      (A) NAME: Prof. Dr. Wolf-Georg Forssmann
      (B) STRASSE: Feodor-Lynen-Str. 31
      (C) ORT: Hannover
      (E) LAND: Deutschland
      (F) POSTLEITZAHL: 30625
   (ii) BEZEICHNUNG DER ERFINDUNG: Zusammensetzung zur Therapie von Diabetes
      mellitus und Fettsucht
   (iii) ANZAHL DER SEQUENZEN: 1
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 28 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 1:
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys
               20                  25

## Beschreibung

Die Erfindung betrifft die Verbindung des Anspruchs 1 sowie ein Arzneimittel enthaltend die erfindungsgemäße Verbindung.

Die hormonelle Regulation der Homöostase des Blutzuckers erfolgt primär durch die pankreatischen Hormone Insulin, Glukagon und Somatostatin. Sie werden in den Langerhansinseln im Pankreas produziert. Diese endokrine Regulation des Blutzuckers steht wiederum unter komplexer Kontrolle durch mit dem Blut zirkulierenden Metaboliten (Glukose, Aminosäuren, Katecholamine, etc.). Obwohl die Insulinsekretion aus dem endokrinen Pankreas überwiegend durch den Blutglukosespiegel stimuliert wird, gibt es auch parakrine Einflüsse durch Hormone wie Glukagon und Somatostatin, auf die Insulinsekretion. Die Modulation der Insulinsekretion in den Inselzellen des Pankreas wird über den Second Messenger zyklisches Adenosinmonophosphat (cAMP) vermittelt.

Der cAMP-Metabolismus der Inselzellen des Pankreas wird auf verschiedenen Stufen reguliert. Zum einen kann die Produktion von cAMP in den pankreatischen Beta-Zellen stimuliert werden, zum anderen kann der Abbau von cAMP in den pankreatischen Beta-Zellen durch verschiedene Phosphodiesterasen stimuliert oder inhibiert werden.

Die WO-A-91/11457 offenbart eine Gruppe von Verbindungen, nämlich modifizierte Varianten von GLP-1-Fragmenten oder amidierte Formen davon. Diese modifizierten Peptide weisen eine mehr oder weniger große Veränderung der nativen Struktur auf.

Es werden erfindungsgemäß vom GLP-1-(7-34)COOH und dem entsprechenden Säureamid abgeleitete Derivate eingesetzt, die folgende allgemeine Formel aufweisen

R-NH-HAEGTFTSDVSSYLEGQAAKEFIAWLVK-CONH₂,

wobei R = H oder eine organische Verbindung mit 1 - 10 C-Atomen ist. Vorzugsweise ist R der Rest einer Carbonsäure. Insbesondere bevorzugt sind die folgenden Carbonsäurereste Formyl-, Acetyl-, Propionyl-, Isopropionyl-, Methyl-, Ethyl-, Propyl-, Isopropyl-, n-Butyl-, sec-Butyl-, tert-Butyl-.

Überraschenderweise besitzt GLP-1-(7-34)amid eine um das 2- bis 3-fach längere Halbwertszeit als das GLP-1-(7-36)amid.

Desweiteren führt die Infusion von GLP-1-(7-34)amid zu einer signifikant höheren Insulinfreisetzung und signifikant stärkeren Reduktion des Glukose-Spiegels als die Infusion von GLP-1-(7-36)amid.

Die erfindungsgemäße Verbindung kann in Kombination mit einem oder mehreren Peptidhormonen, die Einfluß auf die Inselzellsekretion haben, wie z. B. die Hormone der Sekretin / Gastric Inhibitorische Peptid (GIP) / Vasoactive Intestinal Peptide (VIP) / Pituary Adenylate Cyclase Activating Peptide (PACAP) / Glucagon Like Peptide II (GLP-II) / Glicentin / Glukagon Genfamilie und/oder der Adrenomedullin, Amylin, Calcitonin Gene Related Peptide (CGRP) Genfamilie eingesetzt werden.

Vorzugsweise wird die erfindungsgemäße Verbindung als modifiziertes Peptid mit folgenden Modifikationen verwendet:
(a) Substitution der Aminosäure Lysin in Position 26 und/oder 34 durch eine neutrale Aminosäure, Arginin oder eine D Form von Lysin oder Arginin
(b) Substitution von Tryptophan in Position 31 durch eine Oxidation-resistente Aminosäure,
(c) mindestens eine Substitution in folgender Position durch die angegebene Aminosäure :
   Y für V in Position 16;
   K für S in Position 18;
   D für E in Position 21;
   S für G in Position 22;
   R für Q in Position 23;
   R für A in Position 24; und
   Q für K in Position 26;
(d) mindestens eine Substitution in folgender Position durch die angegebene Aminosäure :
   eine neutrale Aminosäure für A in der Position 8;
   eine saure oder neutrale Aminosäure für E in der Position 9;
   eine neutrale Aminosäure für G in der Position 10; und
   eine saure Aminosäure für D in der Position 15; und/oder
(e) Substitution der Aminosäure Histidin in der Position 7 durch eine neutrale Aminosäure oder die D oder die N-acetylierte oder alkylierte Form von Histidin wobei für die angegebenen Substitutionen die Aminosäuren wahlweise in der D- oder L-Form vorliegen und die in der Position 7 substituierte Aminosäure wahlweise in der N-acetylierten oder N-alkylierten Form substituiert ist.

In einer weiteren bevorzugten Ausführungsform weist die erfindungsgemäße Zusammensetzung Modifikationen durch Austausch von Aminosäuren in D- oder L-Form auf. Insbesondere sind solche Modifikationen möglich, bei denen die Aminosäuren Lysin in den Positionen 26 und/oder 34 durch K†, G, S, A, L, I, Q, M, R und Rt und/oder die Aminosäure Tryptophan in der Positionen 31 durch F, V, L, I, A und Y substituiert ist (Das Symbol t bedeutet die D-Form der entsprechenden Aminosäure).

Die oben angegebenen Modifikationen können wahlweise mit mindestens einer Substitution von S für G in Position 22, R in den Positionen 23 und 24 für Q und A, und Q für K in der Position 26 kombiniert wird oder diese Substitutionen zusätzlich mit einer Substitution von D für E in der Position 21 kombiniert werden.

Eine weitere Modifikation betrifft eine Substitution, wobei Alanin in Position 8 durch eine neutrale Aminosäure aus der Gruppe von S, S†, G, C, C†, Sar, A†, beta-ala, und Aib, wobei die in Position 9 für Glutaminsäure substituierte saure oder neutrale Aminosäure aus der Gruppe von E†, D, D†, Cay, T, T†, N, N†, Q, Q†, Cit, MSO, und acetyl-K und wobei die in Position 10 für Gycin substituierte neutrale Aminosäure aus der Gruppe von S, S†, Y, Y†, T, T† N, N†, Q, Q†, Cit, MSO, acetyl-K, F, und F† stammt.

Auch eine Modifikation, bei der die in Position 7 für Histidin substituierte Aminosäure aus der Gruppe von H†, Y, Y†, F; F†, R, R†, Orn, Ornt, M, M†, N-formyl-H, N-formyl-H†, N-acetyl-H, N-acetyl-H†, N-isopropyl-H, N-isopropyl-H†, N-acetyl-K; N-acetyl-K†, P, and P† stammt, kann verwendet werden.

Des weiteren kommt erfindungsgemäß ein Peptid in Betracht, das im Vergleich zu GLP-1(7-34) oder dem C-terminalen Amid eine erhöhte Resistenz gegen Degradation im Plasma besitzt und/oder mindestens eine der folgenden Modifikationen besitzt:
(a) Substitution von Histidin in Position 7 durch die D Form einer neutralen oder sauren Aminosäure oder der D Form von Histidin;
(b) Substitution von Alanin in Position 8 durch die D Form einer Aminosäure, und
(c) Substitution von Histidine in Position 7 durch eine N-acylierte (1-6C) oder N-alkylierte (1-6C) Form einer alternativen Aminosäure oder Histidin, in Frage.

Histidin in Position 7 kann durch eine Aminosäure der Gruppe P†, D†, E†, N†, Q†, L†, V†, I†, und H† substituiert, die D-Aminosäure in Position 8 durch eine Aminosäure der Gruppe P†, V†, L†, I†, und A† substituiert und/oder die D-Aminosäure in Position 8 durch eine alkylierte oder acetylierte Aminosäure der Gruppe P, D, E, N, Q, V, L, I, K, und H substituiert sein.

Es ist für den Fachmann verständlich, dass die Peptidwirkstoffe in phosphorylierter, acetylierter und/oder glycosylierter Form vorliegen können.

Das erfindungsgemäße Arzneimittel enthält eine wirksame Menge der erfindungsgemäßen Verbindung und ist zur Therapie von insulinabhängigen Diabetes mellitus, nicht insulinabhängigen Diabetes mellitus, MODY (maturity-onset diabetes in young people), sekundärer Hyperglykamien im Zusammenhang mit Pankreaserkrankungen (chronische Pankreatitis, Pankreasektomie, Hämochromatose) oder endokrinen Erkrankungen (Akromegalie, Cushing-Syndrom, Phäochromozytom oder Hyperthyreose), medikamentös induzierter Hyperglykämien (Benzuthiadiazin-Saluretika, Diazoxid oder Glukokortikoide), von pathologischer Glukosetoleranz, von Hyperglykämien, von Dyslipoproteinämien, von Fettsucht, von Hyperlipoproteinämien und/oder Hypotonien geeignet.

Die pharmakologisch verträglichen Salze werden in ähnlicher Weise durch Neutralisation der Basen mit anorganischen oder organischen Säuren erhalten. Als anorganische Säuren kommen zum Beispiel Salzsäure, Schwefelsäure, Phosphorsäure oder Bromwasserstoffsäure, als organische Säuren zum Beispiel Carbon-, Sulfo- oder Sulfonsäuren wie Essigsäure, Weinsäure, Milchsäure, Succinsäure, Alginsäure, Benzoesäure, 2-Phenoxybenzoesäure, 2-Acetoxybenzoesäure, Zimtsäure, Mandelsäure, Zitronensäure, Apfelsäure, Salicylsäure, 3-Aminosalicylsäure, Ascorbinsäure, Embonsäure, Nicotinsäure, Isonicotinsäure, Oxalsäure, Aminosäuren, Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphtalin-2-sulfonsäure in Frage.

Zur Herstellung der Arzneimittel wird neben den üblichen Hilfsmitteln, Träger- und Zusatzstoffen eine therapeutisch wirksame Kombination der Einzelsubstanzen oder deren Salze zur Behandlung der genannten Erkrankungen verwendet. Die Dosis des Präparates ist abhängig von Spezies, Körpergewicht, Alter, individuellem Zustand und Applikationsart.

Peptidhaltige Arzneimittel werden in der dem Fachmann bekannten Weise für geeignete Applikationsweisen hergestellt. So kommen insbesondere orale, intravenöse, intramuskuläre, intrakutane, intrathekale und transpulmonale Applikation in Betracht. Die zu verabreichende Dosis für die Verbindung und seine Analoga gemäß Ansprüchen 1-5 beträgt bevorzugt 0,1 µg/kg Körpergewicht bis 10 mg/kg Körpergewicht. Als Applikationsformen kommen auch die in Mizellen und Biopolymere verpackten Peptide in Betracht.

Desweiteren können bekannte Releaseformen, mittels deren die Freisetzung von galenischen Darreichungsformen der Ingredientien dauerhaft oder pulsatorisch erreicht wird, zur Applikation verwendet werden. Dazu gehören vorzugsweise Biopolymere als Träger, Liposomen als Träger oder Infusionspumpen, so dass die Applikation u.a. subkutan, intravenös, peroral, intramuskulär oder transpulmonal durchgeführt werden können.

Feste Arzneiformen können inerte Hilfs- und Trägerstoffe enthalten, wie z.B. Calciumcarbonat, Calciumphosphat, Natriumphosphat, Lactulose, Stärke, Mannit, Alginate, Gelatine, Guar-gummi, Magnesium- oder Aluminiumstearat, Methylcellulose, Talkum, hochdisperse Kieselsäuren, Silikonöl, höhermolekulare Fettsäuren (wie Stearinsäure), Agar-Agar oder pflanzliche oder tierische Fette und Öle, feste hochmolekulare Polymere (wie Polyäthylenglycol); für orale Applikationen geeignete Zubereitungen können gewünschtenfalls zusätzliche Geschmacks- und/oder Süßstoffe enthalten.

Flüssige Arzneiformen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze zur Regelung des osmotischen Drucks oder zur Pufferung und/oder Viskositätsregulatoren enthalten.

Derartige Zusätze sind zum Beispiel Tartrat- und Citrat-Puffer, Ethanol, Komplexbildner (wie Äthylendiamintetraessigsäure und deren nicht-toxische Salze). Zur Regelung der Viskosität kommen hochmolekulare Polymere in Frage wie beispielsweise flüssiges Polyethylenoxid, Carboxymethylcellulosen, Polyvinylpyrrolidone, Dextrane oder Gelatine. Feste Trägerstoffe sind zum Beispiel Stärke, Laktoluse, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglycol).

Ölige Suspensionen für parenterale Anwendungen können vegetabile synthetische oder semisynthetische Öle wie beispielsweise flüssige Fettsäureester mit jeweils 8 bis 22 C-Atomen in den Fettsäureketten, zum Beispiel Palmitin-, Laurin-, Tridecyl-, Margarin-, Stearin-, Arachin-, Myristin-, Behen-, Pentadecyl-, Liol-, Elaidin-, Brasidin-, Eruca-, oder Ölsäure, die mit ein- bis dreiwertigen Alkoholen mit 1 bis 6 C-Atomen wie beispielsweise Methanol, Ethanol, Propanol, Butanol, Pentanol oder deren Isomere, Glycol oder Glycerol verestert sein. Derartige Fettsäureester sind beispielsweise handelsübliche Miglyole, Isopropylmyristat, Isopropylpalmitat, Isopropylstearat, PEG 6-Caprinsäure, Capryl/Caprinsäureester von gesättigten Fettalkoholen, Polyoxyethylenglycaroltrioleate, Ethyloleat, wachsartige Fettsäureester wie künstliches Entenbürzeldrüsenfett, Kokosfettsäure-Isopropylester, Ölsäureoleylester, Ölsäuredecylester, Milchsäureethylester, Dibuthylphthalat, Adipinsäurediisopropylester, Polyolfettsäureester u.a. Ebenso geeignet sind Silikonöle verschiedener Viskosität oder Fettalkohole wie Isotrideoxylalkohol, 2-Octyldodecanol, Cetylstearyl-Alkohol oder Oleylalkohol, Fettsäuren wie beispielsweise Ölsäure. Weiterhin können vegetabile Öle wie Rizinusöl, Mandelöl, Olivenöl, Sesamöl, Baumwollsaatöl, Erdnußöl oder Sojabohnenöl Verwendung finden.

Als Lösungsmittel, Gelbildner und Lösungsvermittler kommen in Frage Wasser oder mit Wasser mischbare Lösungsmittel. Geeignet sind zum Beispiel Alkohole wie beispielsweise Ethanol oder Isopropylalkohol, Benzylalkohol, 2-Octyldodecanol, Polyethylenglykol, Wachse, Methylcelloseive, Celloseive, Ester, Morpholine, Dioxan, Dimethylsulfoxid, Dimethylformamid, Tetrahydrofuran, Cyclohexan.

Als Filmbildner können Celluloseether verwendet werden, die sich sowohl in Wasser als auch in organischen Lösungsmitteln lösen bzw. anquellen können und nach dem Trocknen eine Art Film bilden, wie beispielsweise Hydroxypropylcellulose, Methylcellulose, Ethylzellulose oder lösliche Stärken. Mischformen zwischen Gel- und Filmbildnern sind dadurch ebenfalls möglich. Hier kommen vor allem ionische Makromoleküle zur Anwendung, wie z.B. Natriumcarboxymethylcellulose, Polyacrylsäure, Polymethacrylsäure und deren Salze, Natriumamylopektinsemiglykolat, Alginsäure oder Propylenglykolalginat als Natriumsalz, Gummi arabicum, Xanthan-Gummi, GuarGummi oder Carrageenan.

Als weitere Formulierungshilfsmittel können eingesetzt werden: Glycerin, Paraffin unterschiedlicher Viskosität, Triethanolamin, Collagen, Allantoin, Novantisolsäure, Parfümöle.

Auch die Verwendung von Tensiden, Emulgatoren oder Netzmitteln kann zur Formulierung notwendig sein, wie zum Beispiel von NaLaurylsulfat, Fettalkohlethersulfaten, Di-Na-N-lauryl-β-iminodipropionat, polyoxyethyliertes Rizinusöl, oder Sorbitan-Monooleat, Sorbitan-Monostearat, Cetylalkohol, Lecithin, Glycerinmnonstearat, Polyethylenstearat, Alkylphenolpolyglykolether, Cetyltrimethylammoniumchlorid oder Mono-/Dialkylpolyglykoletherorthophosphorsäure-monoethanolaminsalze.

Stabilisatoren wie Mentmerillenite oder kolloidale Kieselsäure zur Stabilisierung von Emulsionen oder zur Verhinderung des Abbaus der aktiven Substanzen wie Antioxidanzien, beispielsweise Tocopherole oder Buthylhydroxyanisol, oder Konservierungsmittel, wie p-Hydroxybenzoesäureester, können ebenfalls zur Zubereitung der gewünschten Formulierungen gegebenenfalls erforderlich sein.

Die Herstellung, Abfüllung und die Verschließung der Präparate erfolgt unter den üblichen antimikrobiellen und aseptischen Bedingungen. Die Abpackung erfolgt möglichst in separaten Dosiseinheiten zur Erleichterung der Handhabung, auch hier wie bei parenteralen Formen gegebenenfalls aus Stabilitätsgründen durch separate Abpackungen der Wirkstoffe beziehungsweise deren Kombinationen als Lyophilisat, gegebenenfalls mit festen Trägerstoffen und den erforderlichen Lösungsmitteln.

## Patentansprüche

1. Verbindung mit der allgemeinen Formel
R-NH-HAEGTFTSDVSSYLEGQAAKEFIAWLVK-CONH₂,
wobei R = H oder eine organische Verbindung mit 1 - 10 C-Atomen ist.

2. Verbindung gemäß Anspruch 1, wobei R der Rest einer Carbonsäure ist.

3. Verbindung gemäß Anspruch 2, wobei R Formyl-, Acetyl-, Propionyl-, Isopropionyl-, Methyl-, Ethyl-, Propyl-, Isopropyl-, n-Butyl-, sec-Butyl-, tert-Butyl- ist.

4. Verbindung nach mindestens einem der Ansprüche 1 bis 3, wobei mindestens eine in der jeweiligen Position angegebene Aminosäure durch die nachstehende Aminosäure substituiert ist:
eine neutrale Aminosäure für A in der Position 8; ausgewählt aus der Gruppe bestehend aus S, S †, G, C, C †, Sar, β-ala und Aib.
eine neutrale Aminosäure für G in der Position 10; und
eine saure Aminosäure für D in der Position 15.

5. Verbindung nach Anspruch 4, wobei Alanin ausgetauscht ist gegen S, S †, G, C, C †, Sar, β-ala und Aib.

6. Arzneimittel enthaltend eine Verbindung nach mindestens einem der Ansprüche 1 bis 5 zur Therapie von insulinabhängigen Diabetes mellitus, nicht insulinabhängigen Diabetes mellitus, MODY (maturity-onset diabetes in young people), zur Behandlung sekundärer Hyperglykämien im Zusammenhang mit Pankreaserkrankungen (chronische Pankreatitis, Pankreasektomie, Hämochromatose) oder endokrinen Erkrankungen (Akromegalie, Cushing-Syndrom, Phäochromozytom oder Hyperthyreose), zur Behandlung medikamentös induzierter Hyperglykämien (Benzothiadiazin-Saluretika, Diazoxid oder Glukokortikoide), zur Therapie von pathologischer Glukosetoleranz, zur Therapie von Hyperglykämien, zur Therapie von Dyslipoproteinämien, zur Therapie von Fettsucht, zur Therapie von Hyperlipoproteinämien und/oder Hypotonien.

7. Arzneimittel nach Anspruch 6, **dadurch gekennzeichnet, dass** das Arznei mittel in einer Releaseform vorliegt, mittels derer die Freisetzung dauerhaft oder pulsatorisch erreicht wird.

8. Arzneimittel nach Anspruch 6, **dadurch gekennzeichnet, dass** das Arz neimittel für subkutane, intravenöse, perorale, intramuskuläre oder tran spulmonale Applikation geeignet ist.

## Claims

1. A compound of general formula
R-NH-HAEGTFTSDVSSYLEGQAAKEFIAWLVK-CONH₂,
wherein R = H or an organic compound comprising from 1 to 10 carbon atoms.

2. The compound according to claim 1, wherein R is the residue of a carboxylic acid.

3. The compound according to claim 2, wherein R is formyl, acetyl, propionyl, isopropionyl, methyl, ethyl, propyl, isopropyl, n-butyl, sec-butyl, tert-butyl.

4. The compound according to at least one of claims 1 to 3, wherein at least one amino acid stated for the respective position is substituted by the following amino acid:
a neutral amino acid for A in position 8, selected from the group consisting of S, S†, G, C, C†, Sar, beta-ala and Aib;
a neutral amino acid for G in position 10; and
an acidic amino acid for D in position 15.

5. The compound according to claim 4, wherein alanine is substituted by S, S†, G, C, C†, Sar, beta-ala and Aib.

6. A medicament containing a compound according to at least one of claims 1 to 5 for the therapy of insulin-dependent diabetes mellitus, non-insulin-dependent diabetes mellitus, MODY (maturity-onset diabetes in young people), for the treatment of secondary hyperglycemias in connection with pancreatic diseases (chronic pancreatitis, pancreasectomy, hemochromatosis) or endocrine diseases (acromegaly, Cushing's syndrome, pheochromocytoma or hyperthyreosis), for the treatment of drug-induced hyperglycemias (benzothiadiazine saluretics, diazoxide or glucocorticoids), for the therapy of pathologic glucose tolerance, for the therapy of hyperglycemias, for the therapy of dyslipoproteinemias, for the therapy of adiposity, for the therapy of hyperlipoproteinemias and/or hypotensions.

7. The medicament according to claim 6, **characterized in that** said medicament is in a release form by which release is achieved permanently or in a pulsatile way.

8. The medicament according to claim 6, **characterized in that** said medicament is suitable for subcutaneous, intravenous, peroral, intramuscular or transpulmonary administration.

## Revendications

1. Composé de formule générale
R-NH-HAEGTFTSDVSSYLEGQAAKEFIAWLVK-CONH₂,
dans laquelle R = H ou est un composé organique comportant 1 à 10 atomes de carbone.

2. Composé selon la revendication 1, dans lequel R est le résidu d'un acide carboxylique.

3. Composé selon la revendication 2, dans lequel R est un groupe formyle, acétyle, proprionyle, isoproprionyle, méthyle, éthyle, propyle, isopropyle, n-butyle, sec-butyle ou tert-butyle.

4. Composé selon au moins une des revendications 1 à 3, dans lequel au moins un acide aminé indiqué dans sa position respective est substitué par l'acide aminé suivant :
un acide aminé neutre pour A en position 8, choisi dans le groupe comprenant S, S⁺, G, C, C⁺, Sar, β-ala et Aib,
un acide aminé neutre pour G en position 10, et
un acide aminé acide pour D en position 15.

5. Composé selon la revendication 4, dans lequel l'alanine est échangée contre S, S⁺, G, C, C⁺, Sar, β-ala et Aib.

6. Médicament contenant un composé selon au moins une des revendications 1 à 5 destiné à la thérapie du diabète sucré insulino-dépendant, du diabète sucré non insulino-dépendant, du diabète Mason (ou syndrome de MODY - maturity-onset diabete in young people), au traitement des hyperglycémies secondaires liées à des maladies du pancréas (pancréatite chronique, pancréatectomie, hémochromatose) ou des maladies des glandes endocrines (acromégalie, syndrome de Cushing, phéochromocytome ou hyperthyréose), au traitement des hyperglycémies induites par des médicaments (benzothiadazinesalidiurétiques, diazoxyde et glucocorticoïdes), à la thérapie de la tolérance au glucose pathologique, à la thérapie des hyperglycémies, à la thérapie des dyslipoprotéinémies, à la thérapie de l'obésité, à la thérapie des hyperlipoprotéinémies et/ou des hypotonies.

7. Médicament selon la revendication 6, **caractérisé en ce que** le médicament est sous une forme permettant d'obtenir une libération durable ou par pulsations.

8. Médicament selon la revendication 6, **caractérisé en ce qu'**il est approprié pour une application par voie sous-cutanée, intraveineuse, périorale, intramusculaire ou transpulmonaire.
